(19) Europäisches Patentamt / European Patent Office / Office européen des brevets

(11) **EP 4 029 947 A1**

(12) **EUROPEAN PATENT APPLICATION**
published in accordance with Art. 153(4) EPC

(43) Date of publication:
**20.07.2022 Bulletin 2022/29**

(21) Application number: **20862192.0**

(22) Date of filing: **10.09.2020**

(51) International Patent Classification (IPC):
*C12P 21/00* (2006.01)    *A23J 1/00* (2006.01)
*A23L 2/00* (2006.01)    *A23L 5/00* (2016.01)
*C07K 14/415* (2006.01)    *C12G 3/00* (2019.01)
*C12P 21/06* (2006.01)

(52) Cooperative Patent Classification (CPC):
**A23J 1/00; A23L 2/00; A23L 5/00; C07K 14/415;
C12G 3/00; C12P 21/00; C12P 21/06**

(86) International application number:
**PCT/JP2020/034366**

(87) International publication number:
**WO 2021/049591 (18.03.2021 Gazette 2021/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.09.2019 JP 2019166635**

(71) Applicants:
• **Amano Enzyme Inc.
Nagoya-shi
Aichi 460-8630 (JP)**
• **Amano Enzyme U.S.A. Co., Ltd.
Elgin, Illinois 60124 (US)**

(72) Inventor: **OKUDA, Keita
Elgin, Illinois 60124 (US)**

(74) Representative: **Müller-Boré & Partner
Patentanwälte PartG mbB
Friedenheimer Brücke 21
80639 München (DE)**

(54) **METHOD FOR PRODUCING PLANT PROTEIN CONCENTRATE**

(57)    The present invention addresses the problem of providing a means for efficiently producing a plant protein concentrate to be utilized in foods, beverages, etc. The present invention also addresses the problem of raising the utility value of plant proteins and contributing to the improvement of quality in existing uses and the discovery of new uses by making it possible to produce a plant protein concentrate having improved physical properties (especially solubility). The yield of plant protein is improved by treating a plant protein raw material such as peas, soybeans, almonds, etc., with a protein deamidase.

EP 4 029 947 A1

**Description**

TECHNICAL FIELD

The present invention relates to a plant protein concentrate. Particularly, the present invention relates to a method for producing a plant protein concentrate and uses of the product (plant protein concentrate). This application claims priority to Japanese Patent Application No. 2019-166635, filed September 12, 2019, the entire content of which is incorporated herein by reference.

BACKGROUND ART

Lactoproteins, plant proteins, and the like are used for foods and drinks for the purpose of improving nutritive quality, preventing fat separation, improving water-holding capacity, improving shape-retention capacity, improving organoleptic feel in the mouse, or the like. Recently, growing consumer preference for health in particular leads to a considerable increase in demand and consumption of plant proteins. The plant proteins are, for example, prepared as a state of plant protein concentrate with a high protein content by crushing plant protein raw materials such as pulses and grains, and then removing undesired components by alkaline treatment or acid treatment to increase the protein content (see, for example, Non-Patent Documents 1 and 2). Further, a concentrate with an especially high protein content is referred to as a protein isolate, such as a whey protein isolate, which is generally produced by further concentrating and/or purifying the protein concentrate by means of a membrane concentration or the like.

PRIOR ART DOCUMENTS

PATENT DOCUMENT

Patent Document 1: Japanese Patent Laid-open Publication No. 2000-50887

NON-PATENT DOCUMENTS

    Non-Patent Document 1: Int J Mol Sci. 2011; 12(12): 8372-8387.
    Non-Patent Document 2: JOURNAL OF FOOD SCIENCE Vol.46 372 (1981)

SUMMARY OF THE INVENTION

PROBLEMS TO BE SOLVED BY THE INVENTION

It is expected that the demand and consumption of plant proteins will further increase in future, and thus it is desired to supply a plant protein concentrate at a low cost. In addition, the plant proteins have a high potential value due to their characteristics (in particular, their suitability for health-oriented applications), and therefore their widening uses (range of applications) are also expected.
Accordingly, an object of the present invention is to provide a means for efficiently producing a plant protein concentrate used for a food, a drink, and the like. In addition, another object is to accomplish the production of a plant protein concentrate having improved physical properties (in particular, solubility) to improve usefulness of the plant proteins, and therefore contribute to improve their quality for use in conventional applications, create novel applications, and the like.

MEANS FOR SOLVING THE PROBLEM

The inventors have made studies in view of the above objects, and perceived that there are possibilities of increase in yield and improvement in physical properties by an enzyme treatment. As a result, the present inventors have come up with a step of enzyme treatment with a protein deamidase, specifically, treatment of plant protein raw materials with a protein deamidase, for use in a process for manufacturing a plant protein concentrate. The protein deamidase is used for various applications such as food processing (see, for example, Patent Document 1).
When effects of treatment with a protein deamidase were evaluated using four types of plant protein raw materials (peas, chickpea, soybeans, almond, oats, and quinoa), increase in yield of the proteins (increase in the amount obtained) was observed, and an unexpected findings about the relationship between treatment conditions and effects (e.g., increase in yield) was obtained. In addition, when the physical properties of the obtained plant protein concentrate were evaluated, surprisingly, physical properties such as solubility were greatly improved/increased. That is, it was found that the treatment with a protein deamidase had favorable influences not only on yield, but also on physical properties of the plant protein concentrate as a product.

The following invention is principally predicated on the above-described results.

[1] A method for producing a plant protein concentrate, including a step of treating a plant protein raw material with a protein deamidase.
[2] The method according to [1], wherein the plant protein raw material is one or two or more pulses, grains, or seeds selected from the group consisting of peas, chickpea, soybeans, fava bean, lentil, oats, rye, barley, corn, amaranth, sesame, almond, peanut, cashew nut, hazelnut, pecan nut, a macadamia nut, pistachio, walnut, Brazil nut, coconut, chestnut, pine nut, hemp seed, quinoa, and chia seed.
[3] The method according to [1] or [2], wherein the protein deamidase is a protein glutaminase.
[4] The method according to any one of [1] to [3], wherein the protein deamidase is an enzyme derived from a Chryseobacterium genus microorganism.
[5] The method according to [4], wherein the Chryseobacterium genus microorganism is Chryseobacterium proteolyticum.
[6] The method according to any one of [1] to [5], wherein an amount of the protein deamidase used is 0.01 U to 500 U per gram of the plant protein raw material.
[7] The method according to any one of [1] to [6], wherein a concentration of the plant protein raw material for a treatment with the protein deamidase is 10 to 35% (w/w).
[8] The method according to any one of [1] to [7], further including a step of removing components other than proteins after the treatment with the protein deamidase.
[9] The method according to [8], wherein the step of removing components other than proteins further includes the following Steps (1) to (3):

(1) a step of adjusting pH of an enzyme reaction solution and performing an alkaline treatment to separate soluble components after the treatment with protein deamidase,
(2) a step of recovering proteins from separated soluble components by an isoelectric precipitation method, and
(3) a step of subjecting recovered proteins to a neutralization process.

[10] The method according to [9], wherein the alkaline treatment is carried out under a condition with a pH of 8 to 12, and the isoelectric precipitation is carried out under a condition with a pH of 3 to 6.
[11] The method according to [9], wherein the alkaline treatment is carried out under a condition with a pH of 9 to 12.
[12] The method according to [9], wherein the isoelectric precipitation is carried out under a condition with a pH of 4 to 6.
[13] The method according to any one of [9] to [12], further including the following Step (4):
(4) a step of concentrating or drying after the neutralization process.
[14] A plant protein concentrate obtained by the method according to any one of [1] to [13].
[15] A food or drink containing a plant protein concentrate obtained by the method according to any one of [1] to [13].

EMBODIMENTS OF THE INVENTION

1. Method for producing plant protein concentrate

The present invention relates to a method for producing a plant protein concentrate. In the present invention, plant protein raw materials are treated with a protein deamidase to increase the yield of proteins. On the other hand, as supported by the following Examples, according to the present invention, improvements in physical properties of the obtained plant protein concentrate can also be expected.

The term "plant protein concentrate" refers to a composition in which the protein content is increased as compared to that of pre-processed state (i.e., plant protein raw materials) due to extraction or concentration of the proteins. A material that has been concentrated to achieve the protein content of 29 to 89% (w/w) is generally referred to as a protein concentrate. However, the term "protein concentrate" is interpreted in a broad sense in the present invention, and also includes a composition that has been concentrated to achieve the protein content of 90% (w/w) or more, that is, a composition generally referred to as a protein isolate (a protein isolate). In other words, in the present invention, the term "protein concentrate" is used as a term that includes a protein isolate that has a much higher protein content.

The plant protein concentrate such as soy proteins (e.g., soy protein powder) or pea proteins (e.g., pea protein powder) is generally produced by a method in which an alkaline treatment method and an isoelectric precipitation method are used in combination (e.g., see the above-described Non-Patent Documents 1 and 2), a method in which an alkaline treatment method and a membrane separation method are used in combination, or the like. In addition, as described above, a protein isolate is produced through steps, such as membrane concentration and the like, from a protein concentrate. Further, according to the production method of the present invention, a plant protein concentrate having a protein content within a range of 29% to 99% can be obtained, although the protein content may vary according to types/origins of the plant protein raw materials used, protein content, or the like, or the manufacturing process (in particular, whether or not the step includes membrane concentration), manufacturing conditions, and the like.

The production method of the present invention is characterized in that the method "includes a step of treating plant protein raw materials with a protein deamidase". In the present invention, the plant protein raw materials refer to plant materials containing proteins. The plant protein raw materials that are subjected to an enzyme treatment are not particularly limited, and various raw materials that is classified into pulses, grains, or seeds can be used. Specific examples of the plant protein raw materials include peas, chickpea, soybeans, fava bean, lentil, oats, rye, barley, corn, amaranth, sesame, almond, peanut, cashew nut, hazelnut, pecan nut, macadamia nut, pistachio, walnut, Brazil nut, coconut, chestnut, pine nut, hemp seed, quinoa, and chia seed. In addition, materials obtained by processing (e.g., starch extraction or defatting) of the above-described materials, or residues after the processing can be used. Two or more plant protein raw materials can be used in combination.

Crushed materials or pulverized materials (powder) of the plant protein raw materials are generally subjected to the enzyme treatment so that the enzymatic reaction effectively proceeds. Specifically, a protein deamidase is added to a suspension or solution of crushed materials or pulverized materials of plant protein raw materials to cause reactions.

In the present invention, a protein deamidase refers to an enzyme having an effect of directly acting on an amide group of a side chain of an amino acid that constitutes a protein to cause deamidation and release ammonia without cleaving a peptide bond of the protein and crosslinking proteins. Specific examples of the protein deamidase include a protein glutaminase that directly acts on an amide group of a side chain of a glutamine residue contained in a protein to release ammonia and thus converts the glutamine residue into a glutamate residue, and a protein asparaginase that directly acts on an amide group of a side chain of an asparagine residue contained in a protein to release ammonia and thus converts the asparagine residue into an aspartate residue. In the present invention, as a protein deamidase, any one of the protein glutaminase and the protein asparaginase can be used, or both can be used in combination. One preferred example of the protein deamidase used in the present invention is, for example, a protein glutaminase.

The types or origins of the protein deamidase used in the present invention are not particularly limited. Examples of the protein deamidase include protein deamidases derived from Chryseobacterium genus, Flavobacterium genus, Empedobacter genus, Sphingobacterium genus, Aureobacterium genus, or Myroides genus disclosed in the above-described Patent Document 1 (Japanese Patent Laid-open Publication No. 2000-50887), Japanese Patent Laid-open Publication No. 2001-218590, WO 2006/075772, and the like, and commercially available protein glutaminases derived from Chryseobacterium genus. Preferred examples include protein deamidases derived from Chryseobacterium genus, and more preferred examples include protein deamidases derived from Chryseobacterium proteolyticum. Protein glutaminases derived from Chryseobacterium proteolyticum are commercially available as, for example, Protein-glutaminase "Amano" 500 manufactured by Amano Enzyme Inc., and this commercially available products can be used.

As protein deamidases, those prepared from a culture broth of a microorganism that produces protein deamidases can be used. The microorganisms used for the preparation of protein deamidases are not particularly limited, and microorganisms that produce the enzymes and belong to, for example, Chryseobacterium genus, Flavobacterium genus, Empedobacter genus, Sphingobacterium genus, Aureobacterium genus, or Myroides genus can be used. In addition, a microorganism in which a protein deamidase gene is introduced by genetic engineering can be used. Specific examples of the microorganism suitable for the preparation of protein deamidases include Chryseobacterium sp. No. 9670 belonging to Chryseobacterium genus.

For example, protein deamidases can be obtained from a culture broth of the above-described microorganisms or from bacterial cells. That is, secretory proteins can be recovered from a culture broth, and other proteins can be recovered from the interior of bacterial cells. The methods used for the preparation of protein deamidases from a culture broth include publicly-known protein separation and purification methods (e.g., centrifugation, UF concentration, salt precipitation, and various chromatography using, for example, ion exchange resin). For example, a culture broth is centrifuged to remove bacterial cells, and then a desired enzyme can be obtained by using salt precipitation, chromatography, and others in combination. When an enzyme is recovered from the interior of bacterial cells, the bacterial cells are crushed by, for example, pressure treatment or ultrasonication, and then a desired enzyme can be obtained by performing separation or purification as described above. Alternatively, bacterial cells can be preliminary recovered from a culture broth by, for example, filtration or centrifugal treatment, and then the above-described series of steps (crushing, separation, or purification of bacterial cells) can be performed. Powder of the enzyme can be prepared by drying methods such as lyophilization or decompression drying, in which appropriated excipients and drying aids can be used.

In the present application, the activity of a protein deamidase is measured by the following method.

(1) To 1 ml of 0.2 M phosphate buffer (pH 6.5) containing 30 mM Z-X-Gly, 0.1 ml of an aqueous solution containing a protein deamidase is added, incubated at 37°C for 10 minutes, and thereafter 1 ml of 0.4 M TCA solution is added to stop the reaction. A blank is prepared as follows: to a mixture of 1 ml of 0.2 M phosphate buffer (pH 6.5) containing 30 mM Z-X-Gly and 1 ml of 0.4 M TCA solution, 0.1 ml of an aqueous solution containing a protein deamidase is added, and incubated at 37°C for 10 minutes. In the expression "Z-X-Gly", Z represents benzyloxycarbonyl, X represents an L-glutamine residue or an L-asparagine residue, and Gly represents a glycine residue. That is, when X is an L-glutamine residue, "Z-X-Gly" is benzyloxycarbonyl-L-glutaminylglycine, and when X is an L-asparagine residue, "Z-X-Gly" is benzyloxycarbonyl-L-asparaginylglycine. When the protein deamidase to be measured is a protein glutaminase, "Z-X-Gly" in which X is an L-glutamine residue is used, and when the protein deamidase to be measured is a protein asparaginase, "Z-X-Gly" in which X is an L-asparagine residue is used.

(2) The solution obtained in (1) is measured for the amount of ammonia produced in the reaction. The measurement of the amount of ammonia can be carried out using, for example, Ammonia-Test Wako (Wako Pure Chemical Industries, Ltd.). According to a calibration curve that represents the relationship between ammonia concentration and absorbance (630 nm) obtained by using an ammonia standard solution (ammonium chloride), the ammonia concentration in a reaction solution is calculated.

(3) The activity of a protein deamidase is represented by the amount of the enzyme that produces 1 $\mu$mol of ammonia per minute as one unit (U) and calculated using the following formula:

$$\text{Enzyme activity (U/mL)} = \text{ammonia concentration in the reaction solution}$$

$$\text{(mg/L)} \times (1/17.03) \times \text{(reaction fluid volume/amount of the enzyme solution)} \times (1/10) \times$$

$$\text{Df}$$

In the formula, the reaction fluid volume is 2.1, the amount of the enzyme solution is 0.1, and Df represents the dilution factor of the enzyme solution. The numerical value of 17.03 is the molecular weight of ammonia.

The conditions of treatment with a protein deamidase are not particularly limited as long as the treatment is efficient for the effect of the present invention, that is, increase in yield and/or improvement in physical properties of the obtained plant protein concentrate. In preliminary experiments, plant protein raw material concentration, reaction temperature, reaction pH, reaction time, and the amount of the enzyme added (enzyme concentration) can be adjusted, and the optimal reaction conditions depending on the enzyme used can be established.

Without limitation, the plant protein raw material concentration in an untreated liquid (a plant protein raw material solution to be subjected to the treatment with a protein deamidase) is, for example, 5 to 40% (w/w), preferably 10 to 35% (w/w), and more preferably 15 to 30% (w/w). Increase in the plant protein raw material concentration is effective for improving the treatment efficiency and decreasing production cost due to the efficiency improvement. On the other hand, when the plant protein raw material concentration is too high, the solubility of the protein may decrease, leading to possible insufficient treatment. The reaction temperature may be controlled, for example, within a range of 2°C to 70°C, preferably in a range of 5°C to 60°C, and more preferably in a range of 15°C to 50°C. The reaction pH may be controlled, for example, within a range of pH 3 to 10, preferably within a range of pH 4 to 9, and more preferably within a range of pH 5 to 9. Also, the reaction time may be controlled, for example, within a range of 10 minutes to 7 days, preferably within a range of 30 minutes to 3 days, and more preferably within a range of 1 hour to 1 day. Further, the amount of the enzyme added may be controlled, for example, within a range of 0.01 (U/g plant protein raw materials) to 500 (U/g plant protein raw materials), preferably within a range of 0.05 (U/g plant protein raw materials) to 300 (U/g plant protein raw materials), more preferably within a range of 0.1 (U/g plant protein raw materials) to 200 (U/g plant protein raw materials), even more preferably within a range of 0.25 (U/g plant protein raw materials) to 100 (U/g plant protein raw materials), and particularly preferably within a range of 0.25 to 25 (U/g plant protein raw materials). Herein, "U/g plant protein raw materials" is the number of units (U) per gram of plant protein raw materials which is to be treated with a protein deamidase.

After the protein deamidase treatment, when components other than proteins are removed, a plant protein concentrate is obtained. Components other than proteins can be removed by performing extraction and/or concentration of proteins (removing undesired components) by conventional methods (e.g., see the above-described Non-Patent Documents 1 and 2). For example, plant proteins are extracted and/or concentrated by a method in which alkaline treatment (alkali extraction method) and an isoelectric precipitation method are used in combination. When such a method is used, typically, the following Steps (1) to (3) are performed:

(1) a step of adjusting the pH of an enzyme reaction solution and performing alkaline treatment to separate soluble components after the protein deamidase treatment,

(2) a step of recovering proteins from the separated soluble components by an isoelectric precipitation method,
(3) a step of subjecting the recovered proteins to a neutralization process.

Step (1) is a step of separating proteins from undesired components (e.g., dietary fiber and starchy materials) by an alkaline treatment (alkali extraction method), in which the proteins are separated as soluble components. After the enzyme treatment, the pH of the treated solution (enzyme reaction solution) is adjusted to, for example, 8 to 12, preferably the pH is adjusted within a range of 9 to 11, and the solution is treated at, for example, 10°C to 50°C for a predetermined time (e.g., 5 minutes to 24 hours, preferably 10 minutes to 2 hours). For the pH adjustment, NaOH, sodium carbonate, or the like can be used. Raising the pH is effective for increasing protein yield in the alkaline treatment. Thus, for increasing the protein yield, the pH of the enzyme reaction solution is controlled preferably within a range of pH9 to 12, more preferably in a range of pH 9.5 to 11.5, further more preferably in a range of pH10 to 11.

After the alkaline treatment, soluble components (containing proteins) are separated from insoluble components by centrifugal treatment or the like. In centrifugal treatment, soluble components are recovered as the supernatant.

Proteins are recovered from the separated soluble components by an isoelectric precipitation method (Step (2)). For example, when the soluble components are recovered as the supernatant after the centrifugal treatment, the pH of the supernatant (dilution or concentration may be performed before pH adjustment) is adjusted about 3 to 6 to precipitate proteins, and thereafter the precipitate is collected by centrifugal treatment. The pH may be adjusted optimally in consideration of the isoelectric point of the plant protein raw materials used, for example, to pH 3 to 6. Although pH around the isoelectric point is preferably used according to the principle of isoelectric precipitation, to reduce the amount of a pH adjusting agent added, the pH is preferably adjusted within a range of pH 4 to 6, more preferably within a range of pH 4.5 to 6, and still more preferably within a range of pH 4.5 to 5.

After Step (2), a neutralization process is performed (Step (3)). Typically, the precipitate collected in Step (2) is suspended in a suitable solvent (e.g., water is used), and thereafter alkali such as NaOH or sodium carbonate is added to neutralize the suspension. After the neutralization process, if necessary, a treatment by concentration (e.g., membrane concentration, vacuum evaporation, or the like), drying methods (e.g., spray drying, freeze drying, or the like) is performed to obtain a plant protein concentrate. The form of the plant protein concentrate (e.g., powder, granules, liquid, or the like) is not particularly limited.

Although Steps (1) to (3) are typically performed as described above, Step (1) can be omitted (i.e., without performing alkaline treatment), protein extraction and/or concentration can be performed by the isoelectric precipitation in Step (2) (i.e., the enzyme treated solution is subjected to isoelectric precipitation) and the subsequent Step (3). This type of embodiment can be used, for example, in the case where relatively low protein content is acceptable in the plant protein concentrate, leading to a simplified manufacturing process.

2. Use of plant protein concentrate

The plant protein concentrate obtained by the production method of the present invention has a commercial value in itself. On the other hand, it is also useful as a raw material or a material for increasing protein content of various foods, drinks, or the like. Thus, the present application provides foods or drinks containing a plant protein concentrate obtained by the production method of the present invention. Herein, the foods and drinks are not particularly limited. Examples of the foods and drinks include processed seafoods (e.g., tube-shaped fish paste cake, fish paste cake, fish minced and steamed, shredded and dried squid, dried fish, salted fish guts, a fish sausage, foods boiled in soy sauce, canned food, etc.), processed meat products (e.g., ham, bacon, sausage, jerky, corned beef, restructured meat, etc.), processed vegetables (e.g., canned or bottled vegetable, processed tomato, processed mushroom, pickled vegetables, dried vegetables, vegetable boiled down in soy sauce, etc.), noodle and bread (e.g., various noodles, bread, a sweet roll, etc.), processed grains (e.g., cereal, oatmeal, muesli, processed rice, wheat-gluten bread, barley tea, etc.), dairy products (e.g., milk, processed milk, milk beverage, concentrated milk, powdered milk, condensed milk, fermented milk, lactic acid bacteria beverage, butter, cheese, ice cream, etc.), processed fruits (e.g., canned or bottled fruits, jam, marmalade, dried fruits, etc.), confectioneries and deserts (e.g., biscuits, baked pastries, rice confectioneries, fried snacks, Japanese-style uncooked confectioneries, unbaked cakes, semi-perishable confectioneries, Japanese dry confectioneries, candy, chocolate, chewing gum, snack food, frozen desert, etc.), drinks, etc. (e.g., a soft drink, a carbonated drink, a fruit juice, coffee-based drinks, a vegetable juice drink, tea-based drinks, a nonalcoholic drink, alcoholic drinks, etc.), a seasoning agent (e.g., sauce, broth, dressing, sauce, etc.), soup, roux (e.g., curry roux, stew roux, etc.), nutrition-supplement foods and drinks (e.g., protein powder, protein drinks, supplement, nutritional drink, etc.), pet food, and nutritional supplements for pets.

The plant protein concentrate is added and/or admixed to, for example, other raw materials or intermediate processed products in the manufacturing process for a food or drink to which the plant protein concentrate is to be added. Preferably, the plant protein concentrate is added and/or admixed in the final stage of the manufacturing process, that is, after other raw materials are admixed, and the admixture is processed (a stage in which the shape and/or form are those in the final product). Then, optionally, a seasoning agent, a preservative, flavoring agent, an antioxidant, and the like may be added for the purpose of a sterilization treatment, seasoning, or quality preservation. On the other hand, it is also a preferred embodiment that the plant protein concentrate is admixed to a food or drink after the completion of the manufacturing process (i.e., the food or drink has a form of the final product rather than an intermediate product). In this embodiment, the present invention can be applied without changing the manufacturing process of the food or drink.

EXAMPLES

<Production of plant protein concentrate using protein deamidase>

Effectiveness of a protein deamidase for increasing protein yield in a plant protein concentrate was studied. In this study, a protein glutaminase (hereinafter, referred to as "PG") was used as a typical example of protein deamidases.

1. Study on the amount of PG used

(1) Method

Suspensions of 30 g of powder of various plant raw materials (chickpea, peas, and almond) or 15 g of powder of soybeans in 100 mL of water, and suspensions of 30 g of powder of oats or quinoa suspended in 170 mL of water were prepared. To each suspension, a protein glutaminase (Protein-glutaminase "Amano" 500, manufactured by Amano Enzyme Inc.; 500 U/g, protein content: 10 wt% or less) was added in an amount in activity as shown in Table 1, and thereafter the suspension was stirred at 200 rpm for 2 hours at 50°C to perform enzymatic reaction. In this reaction, when powder of oats and quinoa was used, to prevent increase in viscosity by starch contained in the powder, 15 mg of $\alpha$-amylase (KLEISTASE SD80, manufactured by Amano Enzyme Inc.) was added together with the protein glutaminase to perform the enzymatic reaction. Next, the pH of the suspension was adjusted to 10 using 1 N NaOH, and the suspension was subjected to an alkaline treatment with stirring at 200 rpm for 30 minutes at 50°C, and thereafter centrifuged to collect the supernatant. The pH of the collected supernatant was adjusted to a slightly acidic pH using 1 N HCl, and thereafter the precipitate was collected by centrifugation. In this pH adjustment using 1 N HCl, when powder of oats and quinoa was used, the pH was adjusted to 4.5, and when the other powder was used, the pH was adjusted to 4.0. The precipitate was suspended in water, thereafter the suspension was neutralized using 1 N NaOH, and lyophilized to obtain a plant protein concentrate. The protein yield was calculated as a ratio of the weight of the obtained plant protein concentrate to the weight of the plant raw material powder used, and evaluated as a ratio with respect to the protein yield without PG treatment. In this method, the protein yield "without PG treatment" refers to a protein yield when the above-described operation was performed except that PG treatment was not performed.

(2) Results (Table 1)

It is confirmed that the protein yield is increased by the PG treatment. Further, it is also confirmed that the yield is increased by increasing the PG activity. With respect to peas, when the above-described operation was performed under the same conditions except that 750 U of the protein glutaminase was added, the ratio of the protein yield with respect to the protein yield without PG treatment was 124%. Thus, it is confirmed that when the amount of the protein glutaminase added is further increased, the yield is further increased.

[Table 1]

|  | Amount of PG used (U) | Ratio of protein yield with respect to that without PG treatment (%) |
|---|---|---|
| Chickpea | 0 | 100 |
| | 7.5 | 105 |
| | 15 | 108 |
| | 90 | 114 |
| | 150 | 117 |

(continued)

|  | Amount of PG used (U) | Ratio of protein yield with respect to that without PG treatment (%) |
|---|---|---|
| Peas | 0 | 100 |
|  | 7.5 | 112 |
|  | 15 | 117 |
|  | 90 | 122 |
|  | 150 | 118 |
| Soybeans | 0 | 100 |
|  | 3.75 | 103 |
|  | 7.5 | 103 |
|  | 45 | 105 |
|  | 75 | 107 |
| Almond | 0 | 100 |
|  | 7.5 | 126 |
|  | 15 | 124 |
|  | 90 | 130 |
| Oats | 0 | 100 |
|  | 7.5 | 112 |
|  | 15 | 113 |
| Quinoa | 0 | 100 |
|  | 2.25 | 104 |
|  | 15 | 106 |

2. Study on the pH in PG treatment

(1) Method

In 100 mL of water, 30 g of powder of peas was suspended, and thereafter the pH was adjusted to each individual pH using 1 N NaOH or 1 N HCl, and 150 U of a protein glutaminase (Protein-glutaminase "Amano" 500, manufactured by Amano Enzyme Inc.; 500 U/g, protein content: 10 wt% or less) was added to the suspension, and thereafter the suspension was stirred at 200 rpm for 2 hours at 50°C to perform enzymatic reaction. Next, the pH of the suspension was adjusted to 11 using 1 N NaOH, and the suspension was subjected to an alkaline treatment with stirring at 200 rpm for 30 minutes at 50°C, and thereafter centrifuged to collect the supernatant. The pH of the collected supernatant was adjusted to 4.0 using 1 N HCl, and thereafter the precipitate was collected by centrifugation. The precipitate was suspended in water, thereafter the suspension was neutralized using 1 N NaOH, and lyophilized to obtain a plant protein concentrate. The protein yield was calculated as a ratio of the weight of the obtained plant protein concentrate to the weight of the plant raw material powder used, and evaluated as a ratio with respect to the protein yield without PG treatment. In this method, the protein yield "without PG treatment" refers to a protein yield when the above-described operation was performed except that PG treatment was not performed.

(2) Results (Table 2)

It is confirmed that the protein yield is increased by PG treatment at pH 7 to 9.

[Table 2]

| pH in treatment | Ratio of protein yield with respect to that without PG treatment (%) |
|---|---|
| pH7 | 112 |

(continued)

| pH in treatment | Ratio of protein yield with respect to that without PG treatment (%) |
|---|---|
| pH8 | 119 |
| pH9 | 114 |

3. Study on the pH in alkaline treatment

(1) Method

To a suspension of 30 g of powder of each of the various plant raw materials (chickpea, peas) or 15 g of powder of soybeans in 100 mL of water, 150 U (75 U for soybeans) of a protein glutaminase (Protein-glutaminase "Amano" 500, manufactured by Amano Enzyme Inc.; 500 U/g, protein content: 10 wt% or less) was added, and thereafter the suspension was stirred at 200 rpm for 2 hours at 50°C to perform enzymatic reaction. Next, the pH was adjusted to each individual pH using 1 N NaOH, and the suspension was subjected to an alkaline treatment with stirring at 200 rpm for 30 minutes at 50°C, and thereafter centrifuged to collect the supernatant. The pH of the collected supernatant was adjusted to 4.2 using 1 N HCl, and thereafter the precipitate was collected by centrifugation. The precipitate was suspended in water, thereafter the suspension was neutralized using 1 N NaOH, and lyophilized to obtain a plant protein concentrate. The protein yield was calculated as a ratio of the weight of the obtained plant protein concentrate to the weight of the plant raw material powder used, and evaluated as a ratio with respect to the protein yield without PG treatment. In this method, the protein yield "without PG treatment" refers to a protein yield when the above-described operation was performed except that PG treatment was not performed.

(2) Results (Table 3)

It is confirmed that the protein yield is increased by the PG treatment regardless of the pH in the alkaline treatment. Further, it is confirmed that even when the alkaline treatment is performed at pH 11 in which the protein seemed to be completely solubilized, the yield can be increased by the PG treatment. This suggests that proteins that cannot be solubilized by alkaline treatment are solubilized by the PG treatment.

[Table 3]

| | Alkaline treatment | Protein yield (%) | | Ratio of protein yield with respect to that without PG treatment (%) |
|---|---|---|---|---|
| | pH in treatment | Without PG treatment | With PG treatment | |
| Chickpea | pH9 | 16.3 | 17.0 | 104 |
| | pH10 | 17.2 | 18.5 | 108 |
| | pH11 | 17.0 | 17.8 | 105 |
| Peas | pH9 | 3.7 | 5.2 | 141 |
| | pH10 | 4.8 | 6.7 | 138 |
| | pH11 | 7.0 | 9.3 | 133 |
| Soybeans | pH9 | 27.1 | 30.4 | 112 |
| | pH10 | 28.9 | 31.6 | 109 |

4. Study on the pH in acid treatment

(1) Method

To a suspension of 30 g of powder of chickpea or 15 g of powder of soybeans in 100 mL of water, 150 U (75 U for soybeans) of a protein glutaminase (Protein-glutaminase "Amano" 500, manufactured by Amano Enzyme Inc.; 500 U/g, protein content: 10 wt% or less) was added, and thereafter the suspension was stirred at 200 rpm for 2 hours at 50°C to perform enzymatic reaction. Next, the pH of the suspension was adjusted to 10 using 1 N NaOH, and the suspension was subjected to an alkaline treatment with stirring at 200 rpm for 30 minutes at 50°C, and thereafter centrifuged to collect the supernatant. The pH of the collected supernatant was adjusted to each individual pH using 1 N HCl, and thereafter the precipitate was collected by centrifugation. The precipitate was suspended in water, thereafter the suspension was neutralized using 1 N NaOH, and lyophilized to obtain a plant protein concentrate. The protein yield was calculated as a ratio of the weight of the obtained plant protein concentrate to the weight of the plant raw material powder used, and evaluated as a ratio with respect to the protein yield without PG treatment. In this method, the protein yield "without PG treatment" refers to a protein yield when the above-described operation was performed except that PG treatment was not performed.

(2) Results (Table 4)

It is confirmed that the protein yield is increased by the PG treatment regardless of the pH in the acid treatment (treatment in the isoelectric precipitation method). It was expected that the solubility in acidic conditions might be increased by the PG treatment, and thus the yield might be decreased if the pH used was not low. However, a sufficient yield was achieved at a relatively high pH. It should be noted that there was no much difference between the yield at pH 4.5 or higher (especially, at pH 5) and the yield at pH 4.0 or lower.

[Table 4]

| | Acid treatment | Protein yield (%) | | Ratio of protein yield with respect to that without PG treatment (%) |
|---|---|---|---|---|
| | pH in treatment | Without PG treatment | With PG treatment | |
| Chickpea | pH5.0 | 14.8 | 15.9 | 108 |
| | pH4.5 | 14.7 | 15.9 | 108 |
| | pH4.2 | 15.0 | 16.1 | 107 |
| | pH4.0 | 15.1 | 16.0 | 106 |
| | pH3.8 | 15.2 | 15.9 | 104 |
| Soybeans | pH5.0 | 28.0 | 32.7 | 117 |
| | pH4.5 | 28.9 | 33.6 | 116 |
| | pH4.2 | 29.3 | 33.6 | 114 |
| | pH4.0 | 29.6 | 33.3 | 113 |
| | pH3.8 | 29.3 | 34.0 | 116 |
| | pH3.5 | 29.1 | 32.7 | 112 |

5. Evaluation of physical properties of protein concentrate

To a suspension of 30 g of powder of peas in 100 mL of water, a protein glutaminase (Protein-glutaminase "Amano" 500, manufactured by Amano Enzyme Inc.; 500 U/g, protein content: 10 wt% or less) 150 U was added, and thereafter the suspension was stirred at 200 rpm for 2 hours at 50°C to perform enzymatic reaction. Next, the pH was adjusted to 10 using 1 N NaOH, and the suspension was subjected to an alkaline treatment with stirring at 200 rpm for 30 minutes at 50°C, and thereafter centrifuged to collect the supernatant. The pH of the collected supernatant was adjusted to 4.2 using 1 N HCl, and thereafter the precipitate was collected by centrifugation. The precipitate was suspended in water, thereafter the suspension was neutralized using 1 N NaOH, and lyophilized to obtain a pea protein concentrate.

The obtained pea protein concentrate was suspended in water to prepare a suspension having a concentration of 10% (w/w) and a pH of 7.0. The suspension was evaluated for organoleptic feel in the mouse. As a result, the pea protein concentrate with PG treatment had a smooth organoleptic feel in the mouse, whereas the pea protein concentrate without PG treatment had a rough organoleptic feel in the mouse.

Further, the obtained pea protein concentrate was suspended in water to prepare a suspension having a concentration of 10% (w/w) and a pH of 5.5. The suspension was evaluated for solubility. As a result, it was found that the pea protein concentrate with PG treatment had an increased solubility.

From the above-described results, it is also confirmed that PG treatment has an effect to alter the physical properties of the obtained protein concentrate to increase solubility, in addition to the effect of increasing protein yield. Those physical properties are favorable for food and/or drink application, and provide, for example, an improvement in quality or expansion in use.

INDUSTRIAL APPLICABILITY

The present invention provides an efficient method for producing a plant protein concentrate. The plant protein concentrate is used for nutrition-supplement foods and drinks, fortified foods and drinks, and the like. Although examples of protein concentrate/isolate used for foods and drinks include those derived from animal proteins (whey protein isolate, casein protein, and the like), it is expected that the demand for the plant protein concentrate will further grow in future due to, for example, growing consumer preference for health. The present invention meets these demands of the market, and has a great value in use.

The present invention is not limited to the above-described embodiments of the invention and descriptions of Examples in any way. Various modified embodiments fall within the scope of the invention, as long as the embodiments do not depart from the scope of the appended claims and easily occur to those skilled in the art. The entire contents of, for example, all papers, patent application publications, and patents specifically cited in the present specification are incorporated by reference.

**Claims**

1. A method for producing a plant protein concentrate, comprising a step of treating a plant protein raw material with a protein deamidase.

2. The method according to claim 1, wherein the plant protein raw material is one or two or more pulses, grains, or seeds selected from the group consisting of peas, chickpea, soybeans, fava bean, lentil, oats, rye, barley, corn, amaranth, sesame, almond, peanut, cashew nut, hazelnut, pecan nut, macadamia nut, pistachio, walnut, Brazil nut, coconut, chestnut, pine nut, hemp seed, quinoa, and chia seed.

3. The method according to claim 1 or 2, wherein the protein deamidase is a protein glutaminase.

4. The method according to any one of claims 1 to 3, wherein the protein deamidase is an enzyme derived from a Chryseobacterium genus microorganism.

5. The method according to claim 4, wherein the Chryseobacterium genus microorganism is Chryseobacterium proteolyticum.

6. The method according to any one of claims 1 to 5, wherein an amount of the protein deamidase used is 0.01 U to 500 U per gram of the plant protein raw material.

7. The method according to any one of claims 1 to 6, wherein a concentration of the plant protein raw material for a treatment with the protein deamidase is 10 to 35% (w/w).

8. The method according to any one of claims 1 to 7, further comprising a step of removing components other than proteins after the treatment with the protein deamidase.

9. The method according to claim 8, wherein the step of removing components other than proteins further includes the following Steps (1) to (3):

     (1) a step of adjusting pH of an enzyme reaction solution and performing an alkaline treatment to separate soluble components after the treatment with protein deamidase,
     (2) a step of recovering proteins from separated soluble components by an isoelectric precipitation method, and
     (3) a step of subjecting recovered proteins to a neutralization process.

10. The method according to claim 9, wherein the alkaline treatment is carried out under a condition with a pH of 8 to 12, and the isoelectric precipitation is carried out under a condition with a pH of 3 to 6.

11. The method according to claim 9, wherein the alkaline treatment is carried out under a condition with a pH of 9 to 12.

**12.** The method according to claim 9, wherein the isoelectric precipitation is carried out under a condition with a pH of 4 to 6.

**13.** The method according to any one of claims 9 to 12, further comprising the following Step (4):
(4) a step of concentrating or drying after the neutralization process.

**14.** A plant protein concentrate obtained by the method according to any one of claims 1 to 13.

**15.** A food or drink containing a plant protein concentrate obtained by the method according to any one of claims 1 to 13.

| **INTERNATIONAL SEARCH REPORT** | International application No. |
|---|---|
| | PCT/JP2020/034366 |

**A. CLASSIFICATION OF SUBJECT MATTER**
C12P 21/00(2006.01)i; A23J 1/00(2006.01)i; A23L 2/00(2006.01)i; A23L 5/00(2016.01)i; C07K 14/415(2006.01)i; C12G 3/00(2019_01)i; C12P 21/06(2006.01)i
Fl:      C12P21/00 A; A23J1/00 B; A23L2/00; A23L5/00 M; C07K14/415;
         C12G3/00; C12P21/06

According to International Patent Classification (IPC) or to both national classification and IPC

**B. FIELDS SEARCHED**

Minimum documentation searched (classification system followed by classification symbols)
C12P21/00; A23J1/00; A23L2/00; A23L5/00; C07K14/415; C12G3/00; C12P21/06

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2015/133590 A1 (AJINOMOTO CO., INC.) 11 September 2015 (2015-09-11) claims, paragraph [0097] | 1-3, 6-7, 14-15 |
| Y | claims, paragraph [0097] | 4-5, 8-13 |
| Y | JP 2015-524276 A (NESTEC S.A.) 24 August 2015 (2015-08-24) claims, paragraph [0010] | 4-5 |
| A | claims | 1-3, 6-15 |
| Y | JP 8-173052 A (MORINAGA & CO., LTD.) 09 July 1996 (1996-07-09) claims, paragraph [0039], example 1 | 8-13 |
| A | claims, paragraph [0039], example 1 | 1-7, 14-15 |
| A | SUPPAVORASATIT, Inthawoot et al., "Optimization of the Enzymatic Deamidation of Soy Protein by Protein-Glutaminase and Its Effect on the Functional Properties of the Protein", J. Agric. Food Chem., 2011, 59, pp. 11621-11628 abstract | 1-15 |

☐ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 October 2020 (19.10.2020) | 02 November 2020 (02.11.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/034366

| Patent Documents referred in the Report | Publication Date | Patent Family | Publication Date |
|---|---|---|---|
| WO 2015/133590 A1 | 11 Sep. 2015 | US 2017/0044513 A1 claims, paragraph [0186] EP 3130671 A1 | |
| JP 2015-524276 A | 24 Aug. 2015 | US 2015/0257403 A1 claims, paragraph [0012] EP 2882297 A1 | |
| JP 8-173052 A | 09 Jul. 1996 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019166635 A
- JP 2000050887 A
- JP 2001218590 A
- WO 2006075772 A

**Non-patent literature cited in the description**

- *Int J Mol Sci.,* 2011, vol. 12 (12), 8372-8387
- *JOURNAL OF FOOD SCIENCE,* 1981, vol. 46, 372